# EUROPÄISCHE PATENTSCHRIFT

(11) **EP 1 671 587 B1**
(45) Veröffentlichungstag und Bekanntmachung des Hinweises auf die Patenterteilung: **02.09.2009**
(21) Anmeldenummer: 04106745.5
(22) Anmeldetag: 20.12.2004
(51) Int. Cl.: A61B 6/00

(54) **System und Verfahren zum Auslesen von in einer Speicherleuchtstoffschicht gespeicherter Röntgeninformation**
System and method for reading out x-ray information from a storage phosphor layer
Système et procédé de lecture des informations de rayons x d'une couche luminescente d'enregistrement stimulable

(43) Veröffentlichungstag der Anmeldung: 21.06.2006
(73) Patentinhaber: Agfa-Gevaert HealthCare GmbH, 50670 Köln (DE)
(72) Erfinder: Serro, Hannes, 81737, München (DE)

(56) Entgegenhaltungen:
- EP-A- 1 103 219
- EP-A- 1 415 594
- DE-A1- 10 255 958
- US-A1- 2003 142 119
- US-B1- 6 542 579

## Beschreibung

Die Erfindung betrifft ein System sowie ein entsprechendes Verfahren zum Auslesen von in einer Speicherleuchtstoffschicht gespeicherter Röntgeninformation gemäß dem Oberbegriff des Anspruchs 1 bzw. 10.

Eine Möglichkeit zur Aufzeichnung von Röntgenbildern besteht darin, die durch ein Objekt, beispielsweise einen Patienten, hindurch tretende Röntgenstrahlung als latentes Bild in einer so genannten Speicherleuchtstoffschicht zu speichern. Zum Auslesen des latenten Bildes wird die Speicherleuchtstoffschicht mit Stimulationslicht bestrahlt und dabei zur Aussendung von Emissionslicht angeregt. Das Emissionslicht, dessen Intensität dem in der Speicherleuchtstoffschicht gespeicherten Bild entspricht, wird von einem optischen Detektor erfasst und in elektrische Signale umgewandelt. Die elektrischen Signale werden nach Bedarf weiterverarbeitet und schließlich für eine Auswertung, insbesondere zu medizinisch-diagnostischen Zwecken, bereitgestellt, indem sie an einem entsprechenden Ausgabegerät, wie z. B. einem Monitor oder Drucker, ausgegeben werden.

In bekannten Systemen und Verfahren werden vor und/oder nach einer Röntgenaufnahme an einer Dateneingabestation, einer so genannten ID-Station, zusätzliche Informationen zur Identität des Patienten, zur Röntgenaufnahme, zum anschließenden Auslesen des latenten Bildes und/oder Verarbeiten der ausgelesenen Röntgeninformationen eingegeben.

Aus EP 1 415 594 A1 ist bekannt, über einen sog. persönlichen digitalen Assistenten (PDA) eine Identifizierung des jeweiligen Bedieners vorzunehmen sowie radiographische Bedingungen und Anweisungen an ein Radiographiegerät weiterzugeben. Der PDA kann außerdem radiographische Auftragsinformationen von einer Informationsmanagement-Einrichtung empfangen. Die vom PDA empfangenen radiographischen Auftragsinformationen werden einem medizinischen Bild, das aus einer in einer Kassette befindlichen Speicherleuchtstoffplatte ausgelesen wird, zugeordnet und mit diesem in einer Datenbank gespeichert.

In bestimmten Anwendungsfällen, insbesondere bei Notfällen, sind Zeitverzögerungen bei der Eingabe von zusätzlichen Informationen möglichst zu vermeiden. Die aus dem Stand der Technik bekannten Systeme bzw. Verfahren sind für solche Anwendungsfälle somit nur bedingt geeignet.

Es ist Aufgabe der Erfindung, ein System sowie ein entsprechendes Verfahren zum Auslesen von in einer Speicherleuchtstoffschicht gespeicherter Röntgeninformation anzugeben, bei welchem eine möglichst einfache und schnelle Eingabe von Zusatzinformationen ermöglicht wird.

Diese Aufgabe wird gemäß Anspruch 1 bzw. 10 dadurch gelöst, dass zur Eingabe einer Zusatzinformation eine graphische Eingabeeinheit vorgesehen ist, in welcher die Zusatzinformation durch einen Bediener in graphischer Form handschriftlich eingegeben werden kann. Darüber hinaus ist eine Zuordnungseinrichtung zur Zuordnung der in der graphischen Eingabeeinheit handschriftlich eingegebenen Zusatzinformation zur ausgelesenen Röntgeninformation vorgesehen.

Durch die Verwendung einer graphischen Eingabeeinheit wird eine - im Vergleich zu der üblicherweise per Tastatur erfolgende Dateneingabe - einfachere und schnellere Eingabe von Zusatzinformationen ermöglicht. Das erfindungsgemäße System bzw. Verfahren ist daher gerade bei der radiologischen Diagnose von Notfallpatienten von besonderem Vorteil. Darüber hinaus ist die eingebbare graphische Zusatzinformation in Form und Inhalt frei gestaltbar und nicht an spezielle Datenfelder oder Datenformate gebunden. Auf diese Weise können den ausgelesenen Röntgeninformationen Zusatzinformationen, wie z.B. Skizzen oder beliebig gestaltete Zeichen, zugeordnet werden, die bei der Dateneingabe in aus dem Stand der Technik bekannten Systemen nicht berücksichtigt werden können.

Erfindungsgemäß ist die Eingabeeinheit zur handschriftlichen Eingabe der Zusatzinformation durch den Bediener, insbesondere in Form von Text, Bildern oder Symbolen ausgebildet. Die handschriftliche Eingabe stellt eine besonders einfache und direkte Möglichkeit zur Eingabe der Zusatzinformation dar. Beispielsweise kann hierdurch auch eine handschriftliche Signatur durch den Bediener, z.B. in Form einer vollständigen Unterschrift oder eines Namenskürzels, vorgenommen werden.

Vorzugsweise ist die Eingabeeinheit als berührungsempfindliche Eingabeeinheit ausgebildet, in welche die Zusatzinformation durch Berührung der Eingabeeinheit, insbesondere durch Schreib- oder Zeichenbewegungen auf der Eingabeeinheit, eingegeben werden kann. Die Eingabe kann entweder durch einen Finger oder die Hand des Bedieners oder unter Zuhilfenahme eines geeigneten Eingabestiftes erfolgen.

Vorzugsweise ist eine Anzeigeeinheit vorgesehen, welche zum Anzeigen der Zusatzinformation bei deren Eingabe ausgebildet ist. Die Anzeigeeinheit kann dabei entweder separat von der Eingabeeinheit angeordnet sein oder aber mit dieser zusammen in einer kombinierten Anzeige- und Eingabeeinheit integriert sein. Eine solche kombinierte Anzeige- und Eingabeeinheit wird auch als Touch Screen oder Touch Panel bezeichnet. Dies hat den Vorteil, dass der Bediener die Zusatzinformation bei deren Eingabe unmittelbar überprüfen kann.

Die Anzeige- und Eingabeeinheit kann zusätzlich zur Darstellung von Piktogrammen ausgebildet sein, welche unterschiedliche Betriebsmodi der Ausleseeinrichtung und/oder der Eingabeeinheit symbolisieren und durch Berührung der Eingabeeinheit im Bereich der dargestellten Piktogramme ausgewählt werden können. Die Berührung erfolgt hierbei ebenfalls per Finger und/oder Eingabestift. Hierdurch wird zusätzlich die Steuerung der Ausleseeinrichtung bzw. die Menüführung in der Anzeige- und Eingabeeinheit vereinfacht.

In einer Variante der Erfindung ist eine Umwandlungseinrichtung zur Umwandlung der Zusatzinformation von der graphischen Form in eine zeichenorientierte Form vorgesehen und die Zuordnungseinrichtung zur Zuordnung der Zusatzinformation in der zeichenorientierten Form zur ausgelesenen Röntgeninformation ausgebildet. Die Umwandlungseinrichtung wandelt hierbei die handschriftlich eingegebenen Zeichen vorzugsweise in alphanumerische Zeichenfolgen um, welche dann den ausgelesenen Röntgeninformationen zugeordnet werden. Durch die Umwandlung der graphischen Zusatzinformation in eine zeichenorientierte Form wird der Speicherplatzbedarf für die Speicherung der Zusatzinformation reduziert.

Vorzugsweise wird die ausgelesene Röntgeninformation zusammen mit der eingegebenen graphischen Zusatzinformation dargestellt. Die Darstellung erfolgt insbesondere auf einem Bildschirm (Monitor) oder einem Ausdruck (Hardcopy). Die eingegebene Zusatzinformation steht somit zusammen mit der dargestellten Röntgeninformation zur Verfügung und kann unmittelbar zu deren Interpretation, Analyse oder anderen Zwecken herangezogen werden. Dies ist insbesondere bei der Diagnose von Notfallpatienten von Vorteil, bei der neben einer schnellen Eingabe der Zusatzinformation eine möglichst übersichtliche Darstellung der zur Verfügung stehenden zusätzlichen Informationen gefordert ist.

Es ist außerdem bevorzugt, dass die Eingabeeinheit in die Ausleseeinrichtung integriert ist. Hierdurch wird erreicht, dass die graphische Zusatzinformation von einem Bediener eingegebenen werden kann, wenn sich dieser im Bereich der Ausleseeinrichtung aufhält, insbesondere bei der Eingabe bzw. Entnahme der Kassette, in welcher sich die auszulesende Speicherleuchtstoffschicht befindet. Dadurch wird ein separater Gang zu einer Dateneingabestation, beispielsweise einer ID-Station, überflüssig, und die Dateneingabe kann vielmehr sofort an der Ausleseeinrichtung erfolgen.

Weitere Merkmale und Vorteile der Erfindung ergeben sich aus der folgenden Beschreibung bevorzugter Ausführungsformen und Anwendungsbeispiele, wobei Bezug auf die beigefügten Zeichnungen genommen wird.

Es zeigen:
- Fig. 1: ein Ausführungsbeispiel des erfindungsgemäßen Systems;
- Fig. 2: ein Ausführungsbeispiel einer Eingabeeinheit;
- Fig. 3: ein erstes Beispiel für die Eingabe von graphischer Information;
- Fig. 4: ein zweites Beispiel für die Eingabe von graphischer Information; und
- Fig. 5: ein drittes Beispiel für die Eingabe von graphischer Information.

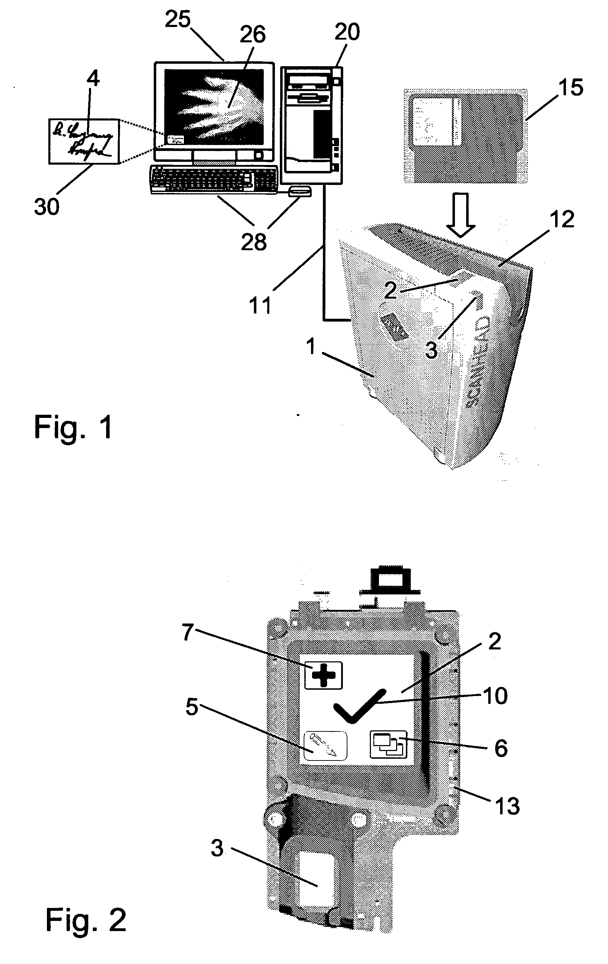

Fig. 1 zeigt ein Ausführungsbeispiel des erfindungsgemäßen Systems, das eine Ausleseeinrichtung 1 zum Auslesen von Röntgeninformationen aus Speicherleuchtstoffschichten umfasst.

Vor dem Auslesen wird eine Kassette 15, in welcher sich eine Speicherleuchtstoffschicht befindet, in eine Kassettenaufnahme 12 der Ausleseeinrichtung 1 eingegeben. In dieser Position wird die Kassette 15 geöffnet und die darin befindliche Speicherleuchtstoffschicht entnommen und in das Innere der Ausleseeinrichtung 1 befördert. Dort wird die Speicherleuchtstoffschicht ausgelesen.

Zum Auslesen ist vorzugsweise ein so genannter Zeilenscanner vorgesehen, bei welchem jeweils eine ganze Zeile der Speicherleuchtstoffschicht mit Stimulationslicht bestrahlt wird und das hierbei erzeugte Emissionslicht mit einem zeilenförmigen Detektorarray erfasst wird. Einzelheiten zum Aufbau eines solchen Zeilenscanners können beispielsweise aus der US 6,373,074 B1 entnommen werden. Alternativ kann hierfür auch ein so genannter Flying Spot-Scanner eingesetzt werden, wie er beispielsweise in der US 6,501,088 B1 beschrieben ist.

Nach dem Auslesevorgang werden in der Speicherleuchtstoffschicht verbleibende Restinformationen durch Bestrahlung der Speicherleuchtstoffschicht mit Licht einer geeigneten Spektralverteilung und Intensität gelöscht. Nach dem Löschen wird die Speicherleuchtstoffschicht wieder zurück in die Kassette 15 transportiert und kann von dem Bediener entnommen und für weitere Röntgenaufnahmen bereitgestellt werden.

Die Ausleseeinrichtung 1 weist eine Statusanzeige 3 zur Anzeige des jeweiligen Betriebszustands auf. Leuchtet oder blinkt die Statusanzeige 3 beispielsweise grün, so wird signalisiert, dass die Ausleseeinrichtung 1 betriebsbereit bzw. vorübergehend mit dem Auslesen und Löschen einer Speicherleuchtstoffschicht beschäftigt ist. Leuchtet oder blinkt dagegen die Statusanzeige 3 rot, so wird das Vorliegen eines Fehlers bzw. die Durchführung eines Start- oder Selbsttestprogramms signalisiert.

Neben der Statusanzeige 3 weist die Ausleseeinrichtung 1 eine Eingabeeinheit 2 auf, über welche durch den Bediener Zusatzinformationen eingegeben werden können. Die Eingabeeinheit 2 ist hierbei erfindungsgemäß als graphische Eingabeeinheit ausgebildet, so dass die Zusatzinformation durch den Bediener in graphischer Form eingegeben werden kann. Die Eingabe der Zusatzinformation erfolgt dabei vorzugsweise unmittelbar vor oder nach dem Auslesen oder aber auch während des Auslesens der Speicherleuchtstoffschicht in der Ausleseeinrichtung 1.

Die eingegebene Zusatzinformation wird erfindungsgemäß den jeweils ausgelesenen Röntgeninformationen zugeordnet. Dies erfolgt vorzugsweise durch eine Übertragung sowohl der ausgelesenen Röntgeninformation als auch der eingegebenen Zusatzinformation über eine Datenleitung 11 zu einem Computersystem 20, in welchem ein Speicher zur Speicherung dieser Informationen vorgesehen ist.

Die Zuordnung der Zusatzinformation zu den jeweiligen Röntgeninformationen kann beispielsweise durch Speichern der Zusatzinformation und der zugehörigen Röntgeninformationen in einem gemeinsamen Datenfeld im Speicher des Computersystems 20 erfolgen. Alternativ oder zusätzlich können beide Informationen aber auch in unterschiedlichen Datenfeldern gespeichert werden, wobei eine Zuordnung beispielsweise durch geeignete Adresszeiger erreicht wird.

Das erfindungsgemäße System weist darüber hinaus einen Bildschirm 25 auf, auf dem die ausgelesene Röntgeninformation in Form eines Röntgenbildes 26 zusammen mit der eingegebenen Zusatzinformation 4 dargestellt wird. Im gezeigten Beispiel wird die Zusatzinformation 4 in einem gesonderten Darstellungsfeld 30 im linken unteren Bereich des dargestellten Röntgenbildes 26 wiedergegeben. Zur Verbesserung der Anschaulichkeit sind das Darstellungsfeld 30 und die darin dargestellte Zusatzinformation 4 zusätzlich vergrößert dargestellt. Die Größe und Anordnung des Darstellungsfeldes 30 für die eingegebene Zusatzinformation 4 im Röntgenbild 26 kann vom Bediener mittels geeigneter Bedienelemente 28, wie z. B. Tastatur und/oder Computermaus, beliebig verändert werden.

Fig. 2 zeigt ein Ausführungsbeispiel einer Eingabeeinheit 2, die zusammen mit der Statusanzeige 3 auf einer Platine 13 angeordnet ist. Durch die Kombination der beiden Komponenten auf einer gemeinsamen Platine 13 wird die Integration sowohl der Eingabeeinheit 2 als auch der Statusanzeige 3 in das Gehäuse der Ausleseeinrichtung 1 sowie die schaltungstechnische Realisierung vereinfacht.

Die Eingabeeinheit 2 ist in dem dargestellten Beispiel mit einer Anzeigeeinheit kombiniert und bildet zusammen mit dieser eine Anzeige- und Eingabeeinheit, welche auch als Touch Screen oder Touch Panel bezeichnet wird.

Im Bereich der Eingabeeinheit 2 werden in der Anzeigeeinheit unterschiedliche Piktogramme 5, 6 und 7 sowie ein Symbol 10 angezeigt. Durch Berührung der Eingabeeinheit 2 im Bereich eines Piktogramms 5, 6 bzw. 7 wird die dem jeweiligen Piktogramm 5, 6 bzw. 7 zugeordnete Funktion ausgewählt und ausgeführt.

So wird z. B. durch Berühren der Eingabeeinheit 2 im Bereich des ersten Piktogramms 7 ein Betriebsmodus ausgewählt, in welchem die in der Kassette 15 befindliche Speicherleuchtstoffschicht sofort in die Ausleseeinrichtung 1 eingegeben und ausgelesen werden kann, ohne vorher oder anschließend die Eingabe von Daten zum Patienten, zur Röntgenaufnahme und/oder Weiterverarbeitung der ausgelesenen Röntgeninformation zu erfordern. Dieser Betriebsmodus wird auch als Emergency Case bezeichnet, da er insbesondere bei Notfällen, in denen keine Zeit für die Eingabe der o.g. Daten ist, Verwendung findet.

Durch Betätigen des zweiten Piktogramms 6 kann eine Übersicht über weitere Funktionen bzw. Betriebsmodi der Eingabeeinrichtung 2 und/oder Ausleseeinrichtung 1 abgerufen werden.

Durch Anwählen des dritten Piktogramms 5 wird die Eingabeeinheit 2 in einen graphischen Eingabemodus versetzt, in welchem der Bediener Zusatzinformationen in graphischer Form eingeben kann.

Das dargestellte Symbol 10 in Form eines Hakens zeigt die allgemeine Betriebsbereitschaft der Ausleseeinrichtung 1 an.

Die aktive Anzeigefläche der Eingabeeinheit 2 weist eine seitliche Abmessung auf, welche typischerweise zwischen 50 und 100 mm liegt. Die Anzahl der Bildpunkte (Pixel) liegt typischerweise zwischen 20.000 und 100.000. Typische Abmessungen eines Bildpunktes liegen im Bereich zwischen 0,2 und 0,5 mm. Im dargestellten Beispiel der Fig. 2 beträgt die aktive Anzeigefläche 56x56 mm, wobei die einzelnen Bildpunkte eine Fläche von 0,35x0,35 mm abdecken. Insgesamt umfasst die aktive Anzeigefläche somit 160x160=25.600 Pixel. Jedem Bildpunkt der Anzeigeeinheit ist ein berührungsempfindlicher Bereich der Eingabeeinheit 2 zugeordnet.

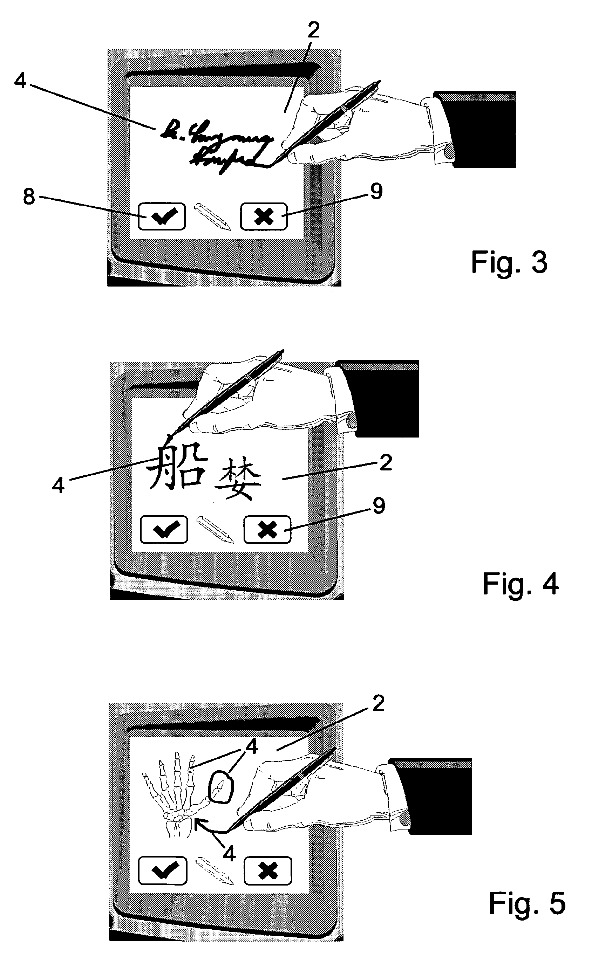

Die Fig. 3 bis 5 zeigen Beispiele für die Eingabe von graphischer Information über die Eingabeeinheit 2, während sich diese im graphischen Eingabemodus befindet.

In Fig. 3 wird die Zusatzinformation 4 in Form eines handschriftlichen Textes oder einer Unterschrift eingegeben. Die Eingabe erfolgt hierbei - wie angedeutet - unter Zuhilfenahme eines Eingabestiftes.

Durch Berühren eines vierten Piktogramms 9 kann die aktuell eingegebene graphische Zusatzinformation wieder gelöscht werden und ggf. erneut eingegeben werden.

Durch Berühren des fünften Piktogramms 8 wird der Eingabemodus beendet. Die Anzeige der Eingabeeinheit 2 wird dann wieder in den in Fig. 2 dargestellten Zustand zurückgesetzt. Die eingegebene graphische Zusatzinformation kann nun zusammen mit der ausgelesenen Röntgeninformation gespeichert werden, vorzugsweise in einem Speicher des Computersystems 20 (siehe Fig. 1).

Wie das in Fig. 4 dargestellte zweite Beispiel zeigt, kann die graphische Zusatzinformation 4 auch in Form beliebiger nationaler Schriftzeichen und/oder Symbole erfolgen. Die Anzeige- und Eingabeeinheit ist vorzugsweise mit einer Funktion versehen, in welcher die angezeigten Piktogramme, Symbole oder Textinformationen beliebig umgestaltet und somit an besondere Anwendungsfälle oder die jeweilige Landessprache angepasst werden können. So kann beispielsweise das vierte Piktogramm 9 in ein entsprechendes asiatisches Schriftzeichen für "Löschen" geändert werden.

Wie das dritte Beispiel in Fig. 5 zeigt, kann die Zusatzinformation 4 auch in Form eines Bildes und/oder in Form von Symbolen oder Hervorhebungen zu einem jeweils angezeigten Bild eingegeben werden.

Bei den dargestellten Ausführungsbeispielen wird die graphische Eingabeeinheit 2 so betrieben, dass sie sowohl zur einfachen Steuerung der Ausleseeinrichtung 1 und/oder der Eingabeeinheit 2 durch Berühren von Piktogrammen als auch zur einfachen und schnellen handschriftlichen Eingabe einer graphischen Zusatzinformation dient. Hierdurch ist sie für die radiologische Diagnose von Notfallpatienten besonders geeignet.

## Patentansprüche

1. System zum Auslesen von in einer Speicherleuchtstoffschicht gespeicherter Röntgeninformation mit
- einer Ausleseeinrichtung (1) zum Auslesen der in der Speicherleuchtstoffschicht gespeicherten Röntgeninformation und
- einer Eingabeeinheit (2) zur Eingabe einer Zusatzinformation durch einen Bediener, wobei die Eingabeeinheit (2) als graphische Eingabeeinheit ausgebildet ist,
- einem Computersystem (20) mit einem Speicher
- einer Zuordnungseinrichtung zur Zuordnung der durch den Bediener in der Eingabeeinheit (2) in graphischer Form handschriftlich eingegebenen Zusatzinformation (4) zur ausgelesenen Röntgeninformation,
**dadurch gekennzeichnet, dass**
die Zuordnung dadurch erfolgt, dass die ausgelesene Röntgeninformation und die handschriftlich eingegebene Zusatzinformation (4) in graphischer Form im Speicher des Computersystems (20) gespeichert werden.

2. System nach Anspruch 1, wobei die Eingabeeinheit (2) zur handschriftlichen Eingabe der Zusatzinformation (4) in Form von Text, Bildern, Symbolen oder einer Signatur ausgebildet ist.

3. System nach Anspruch 1 oder 2, wobei die Eingabeeinheit (2) als berührungsempfindliche Eingabeeinheit ausgebildet ist, in welche die Zusatzinformation durch Berührung der Eingabeeinheit (2), insbesondere durch Schreib- oder Zeichenbewegungen auf der Eingabeeinheit (2), eingegeben werden kann.

4. System nach einem der vorangehenden Ansprüche, wobei eine Anzeigeeinheit zum Anzeigen der Zusatzinformation (4) bei deren Eingabe vorgesehen ist.

5. System nach Anspruch 3 und 4, wobei die Anzeigeeinheit und die Eingabeeinheit (2) in einer Anzeige- und Eingabeeinheit integriert sind, welche insbesondere als Touch Screen ausgebildet ist.

6. System nach Anspruch 5, wobei die Anzeige- und Eingabeeinheit zur Darstellung von Piktogrammen (5 bis 9) ausgebildet ist, welche unterschiedliche Betriebsmodi der Ausleseeinrichtung (1) und/oder der Eingabeeinheit (2) symbolisieren und durch Berührung der Eingabeeinheit (2) ausgewählt werden können.

7. System nach einem der vorangehenden Ansprüche, wobei eine Umwandlungseinrichtung zur Umwandlung der Zusatzinformation (4) von der graphischen Form in eine zeichenorientierte Form vorgesehen ist und die Zuordnungseinrichtung zur Zuordnung der Zusatzinformation in der zeichenorientierten Form zur Röntgeninformation ausgebildet ist.

8. System nach einem der vorangehenden Ansprüche, wobei eine Darstellungseinrichtung, insbesondere ein Bildschirm (25) oder ein Drucker, zu einer Darstellung (26, 30) der ausgelesenen Röntgeninformation zusammen mit der eingegebenen Zusatzinformation (4) vorgesehen ist.

9. System nach einem der vorangehenden Ansprüche, wobei die Eingabeeinheit (2) in die Ausleseeinrichtung (1) integriert ist.

10. Verfahren zum Auslesen von in einer Speicherleuchtstoffschicht gespeicherter Röntgeninformation mit folgenden Schritten:
- Eingabe einer Zusatzinformation in eine Eingabeeinheit (2) durch einen Bediener und
- Auslesen der in der Speicherleuchtstoffschicht gespeicherten Röntgeninformation,
wobei:
- die Zusatzinformation (4) durch den Bediener in graphischer Form in die Eingabeeinheit (2) handschriftlich eingegeben wird und
- die in der Eingabeeinheit (2) in graphischer Form handschriftlich eingegebene Zusatzinformation der ausgelesenen Röntgeninformation zugeordnet wird,
**dadurch gekennzeichnet, dass**
die ausgelesene Röntgeninformation und die handschriftlich eingegebene Zusatzinformation (4) in graphischer Form in einem Speicher eines Computersystems (20) gespeichert werden.

11. Verfahren nach Anspruch 10, wobei die handschriftliche Eingabe der Zusatzinformation (4) in die Eingabeeinheit (2) in Form von Text, Bildern, Symbolen oder einer Signatur erfolgt.

12. Verfahren nach Anspruch 10 oder 11, wobei die Zusatzinformation (4) durch Berührung der Eingabeeinheit (2), insbesondere durch Schreib- oder Zeichenbewegungen auf der Eingabeeinheit (2), eingegeben wird.

13. Verfahren nach einem der Ansprüche 10 bis 12, wobei die Zusatzinformation (4) bei deren Eingabe angezeigt wird.

14. Verfahren nach Anspruch 13, wobei Piktogramme (5 bis 9), welche unterschiedliche Betriebsmodi der Ausleseeinrichtung (1) und/oder der Eingabeeinheit (2) symbolisieren, angezeigt und durch Berührung der Eingabeeinheit (2) ausgewählt werden.

15. Verfahren nach einem der Ansprüche 10 bis 14, wobei die Zusatzinformation von der graphischen Form in eine zeichenorientierte Form umgewandelt wird, in welcher sie der Röntgeninformation zugeordnet wird.

16. Verfahren nach einem der Ansprüche 10 bis 15, wobei die ausgelesene Röntgeninformation (26) zusammen mit der eingegebenen Zusatzinformation (4, 30), insbesondere auf einem Bildschirm (25) oder einem Ausdruck, dargestellt wird.

## Claims

1. A system for reading out X-ray information stored in a storage phosphor layer having
- a read-out device (1) for reading out the X-ray information stored in the storage phosphor layer and
- an input unit (2) for the inputting of additional information by an
- operator, the input unit (2) being in the form of a graphical input unit,
- a computer system (20) with a memory,
- an allocation device for allocating the additional information (4) inputted in hand-writing by the operator into the input unit (2) in graphic form to the read out X-ray information,
**characterised in that**
the allocation is implemented by the read out X-ray information and the additional information (4) inputted in hand-writing being stored in the memory of the computer system (20) in graphic form.

2. The system according to Claim 1, the input unit (2) being designed for the inputting in hand-writing of the additional information (4) in the form of text, images, symbols or a signature.

3. The system according to Claim 1 or 2, the input unit (2) being designed as a touch-sensitive input unit into which the additional information can be inputted by touching the input unit (2), in particular by writing or sign movements on the input unit (2).

4. The system according to any of the preceding claims, a display unit being provided for displaying the additional information (4) when the latter is inputted.

5. The system according to Claims 3 and 4, the display unit and the input unit (2) being integrated into a display and input unit which is in particular in the form of a touch screen.

6. The system according to Claim 5, the display and input unit being designed to display pictograms (5 to 9) which symbolise different operating modes of the read-out device (10) and/or the input unit (2) and can be selected by touching the input unit (2).

7. The system according to any of the preceding claims, a conversion device being provided for converting the additional information (4) from the graphic form into a sign-orientated form, and the allocation device for allocating the additional information being designed in the sign-orientated form for the X-ray information.

8. The system according to any of the preceding claims, a display device, in particular a screen (25) or a printer, being provided for a display (26, 30) of the read out X-ray information together with the inputted additional information (4).

9. The system according to any of the preceding claims, the input unit (2) being integrated into the read-out device (1).

10. A method for reading out X-ray information stored in a storage phosphor layer having the following steps:
- the inputting of additional information into an input unit (2) by an operator and
- reading out the X-ray information stored in the storage phosphor layer,
- the additional information (4) being inputted in hand-writing by the operator into the input unit (2) in graphic form, and
- the additional information inputted in hand-writing into the input unit (2) in graphic form being allocated to the read out X-ray information,
**characterised in that**
- the read out X-ray information and the additional information (4) inputted in hand-writing is stored in graphic form in a memory of a computer system (20).

11. The method according to Claim 10, the inputting of the additional information (4) in hand-writing into the input unit (2) being in the form of text, images, symbols or a signature.

12. The method according to Claim 10 or 11, the additional information (4) being inputted by touching the input unit (2), in particular by writing or sign movements on the input unit (2).

13. The method according to any of Claims 10 to 12, the additional information (4) being displayed when the latter is inputted.

14. The method according to Claim 13, pictograms (5 to 9), which symbolise different operating modes of the read-out device (1) and/or of the input unit (2), being displayed and selected by touching the input unit (2).

15. The method according to any of Claims 10 to 14, the additional information being converted from the graphic form into a sign-orientated form in which it is allocated to the X-ray information.

16. The method according to any of Claims 10 to 15, the read out X-ray information (26) being displayed together with the inputted additional information (4, 30), in particular on a screen (25) or a printout.

## Revendications

1. Système permettant de lire une information radiographique enregistrée dans une couche de phosphore, comportant
- un dispositif de lecture (1) destiné à lire l'information radiographique enregistrée dans la couche de phosphore et
- une unité de saisie (2) permettant à un opérateur de saisir une information supplémentaire, l'unité de saisie (2) consistant en une unité de saisie graphique,
- un système informatique (20) possédant une mémoire,
- une unité d'affectation destinée à affecter à l'information radiographique lue ladite information supplémentaire (4) saisie par l'opérateur de façon manuscrite, sous forme graphique dans l'unité de saisie (2),
**caractérisé en ce que**
l'affectation prend effet **en ce que** l'information radiographique lue et l'information supplémentaire (4) saisie à la main sont enregistrées sous forme graphique dans la mémoire du système informatique (20).

2. Système selon la revendication 1, dans lequel l'unité de saisie (2) est conçue pour la saisie manuscrite de l'information supplémentaire (4), sous la forme d'un texte, d'images, de symboles ou d'une signature.

3. Système selon la revendication 1 ou 2, dans lequel l'unité de saisie (2) est conçue pour être sensitive, l'information supplémentaire pouvant être saisie en touchant l'unité de saisie (2), notamment en exerçant des mouvements d'écriture ou de tracé sur ladite unité de saisie (2).

4. Système selon l'une des revendications précédentes, dans lequel il est prévu une unité d'affichage destinée à afficher l'information supplémentaire (4) lors de sa saisie.

5. Système selon les revendications 3 et 4, dans lequel l'unité d'affichage et l'unité de saisie (2) sont intégrées au sein d'une unité d'affichage et de saisie conçue notamment sous la forme d'un écran tactile.

6. Système selon la revendication 5, dans lequel l'unité d'affichage et de saisie est conçue pour la représentation de pictogrammes (5 à 9) symbolisant différents modes de fonctionnement du dispositif de lecture (1) et/ou de l'unité de saisie (2) et susceptibles d'être sélectionnés en touchant l'unité de saisie (2).

7. Système selon l'une des revendications précédentes, dans lequel il est prévu un dispositif de conversion destiné à convertir l'information supplémentaire (4) de la forme graphique en une forme orientée caractère et dans lequel l'unité d'affectation est conçue pour affecter l'information supplémentaire sous la forme orientée caractère à l'information radiographique.

8. Système selon l'une des revendications précédentes, dans lequel il est prévu un dispositif de représentation, notamment un écran (25) ou une imprimante, permettant la représentation (26, 30) de l'information radiographique lue conjointement à l'information supplémentaire (4) saisie.

9. Système selon l'une des revendications précédentes, dans lequel l'unité de saisie (2) est intégrée dans le dispositif de lecture (1).

10. Procédé permettant de lire une information radiographique enregistrée dans une couche de phosphore comportant les étapes suivantes :
- saisie, par un opérateur, d'une information supplémentaire dans une unité de saisie (2) et
- lecture de l'information radiographique enregistrée dans la couche de phosphore,
dans lequel
- l'information supplémentaire (4) est saisie par l'opérateur sous une forme graphique dans l'unité de saisie (2) et
- l'information supplémentaire saisie à la main dans l'unité de saisie (2) sous forme graphique est affectée à l'information radiographique lue,
**caractérisé en ce que** l'information radiographique lue et l'information supplémentaire (4) saisie à la main sont enregistrées sous forme graphique dans une mémoire d'un système informatique (20).

11. Procédé selon la revendication 10, dans lequel l'entrée manuscrite de l'information supplémentaire (4) dans l'unité de saisie (2) s'effectue sous la forme d'un texte, d'images, de symboles ou d'une signature.

12. Procédé selon la revendication 10 ou 11, dans lequel l'information supplémentaire (4) est saisie en touchant l'unité de saisie (2), notamment en exerçant des mouvements d'écriture ou de tracé sur ladite unité de saisie (2).

13. Procédé selon l'une des revendications 10 à 12, dans lequel l'information supplémentaire (4) s'affiche lors de sa saisie.

14. Procédé selon la revendication 13, dans lequel des pictogrammes (5 à 9) symbolisant différents modes de fonctionnement du dispositif de lecture (1) et/ou de l'unité de saisie (2) sont affichés, et sont choisis en touchant l'unité de saisie (2).

15. Procédé selon l'une des revendications 10 à 14, dans lequel l'information supplémentaire est convertie de la forme graphique en une forme orientée caractère, sous laquelle elle est affectée à l'information radiographique.

16. Procédé selon l'une des revendications 10 à 15, dans lequel l'information radiographique (26) lue est représentée conjointement à l'information supplémentaire (4, 30) saisie, notamment sur un écran (25) ou sous forme imprimée.
